# EUROPEAN PATENT SPECIFICATION

(11) **EP 0 488 470 B1**
(45) Date of publication and mention of the grant of the patent: **28.05.1997**
(21) Application number: 91203080.6
(22) Date of filing: 25.11.1991
(51) Int. Cl.: C12N 15/02, C12P 21/08

(54) **Method for the production of antibodies**
Verfahren zur Herstellung von Antikörpern
Procédé de production d'anticorps monoclonaux

(30) Priority: 26.11.1990 EP 90203124
(43) Date of publication of application: 03.06.1992
(73) Proprietor: Akzo Nobel N.V., 6824 BM Arnhem (NL)
(72) Inventor: Steenbakkers, Petrus Gerardus Antonius, NL-5469 VL Erp (NL)
(74) Representative: Hermans, Franciscus G.M.

(56) References cited:
- EP-A- 0 131 878
- EUROPEAN JOURNAL OF IMMUNOLOGY, vol. 17, 1987,pages 887-892; L. WEN
- IMMUNOLOGICAL REVIEWS, vol. 99, 1987, pages 281-297; R.H. ZUBLER

## Description

The invention relates to a method for the production of monoclonal antibodies.
The standard procedure for the generation of monoclonal antibodies as originally described by Köhler and Milstein in 1975 (1) involves the fusion of sensitized murine spleen cells with murine myeloma cells in the presence of polyethyleneglycol (PEG). However, this method is rather inefficient. Usually, at best only one B-cell at 2.10⁵ spleen cells fuses successfully with a myeloma cell to yield a stable growing hybridoma. This means that 99,9995% of the cells are lost.

In 1982 (2), Zimmermann and co-workers introduced electrofusion as an efficient alternative for PEG-induced cell fusion. In recent years, this technique has been further explored for the generation of antibody producing hybridomas of both murine and human origin. The technique is based on the observation that close membrane contact between two cells can be established in an alternating electric field (dielectrophoresis). Subsequent exposure to a field pulse of high intensity and short duration leads to temporary permeabilisation of the cell membrane. After this short pulse, cells are allowed to reseal in an alternating electric field. During resealing, cells that are in close contact may create a cytoplasmic bridge leading to cell fusion. Electrofusion frequencies up to 1:2000 are reported (3).

Still, only spleen cells can be used as the source of B-cells, because fusions with lymph node cells or peripheral blood cells only yield too limited a number of hybridomas or are not possible at all. The same holds true for the limited amount of cells resulting from in vitro immunization or selection experiments.

Another attempt to generate monoclonal antibodies involves the PCR-technique for Ig-genes.
The polymerase chain reaction (PCR) technique (4) has been used for specific amplification of a defined fragment of DNA in vitro. It involves repeated rounds of extension from two specific primers for regions at each end of the gene to be amplified.
Recently, the technique has been adapted for amplification of rearranged immunoglobulin variable gene fragments using mRNA from hybridoma cells as starting material (5). On account of the diversity of the family of Ig molecules, no universal, specific primers could be constructed. Therefore, Larrick et al. (6, 7, 8) have designed degenerate mixtures of oligonucleotide primers from known leader sequences of human heavy and light chains and from sequences corresponding to the N-terminal part of human immunoglobulin constant regions. The same was done for mouse immunoglobulin sequences by Le Boeuf et al. (9). Thus it will be possible to amplify, sequence and express the variable region sequences of heavy and light chains from any human or murine immunoglobulin.
The PCR technique for cloning of complete Ig genes in mammalian cells, however, will not be suitable to generate a large number of antibody producing cell lines because, in contrary to the generation of hybridomas, expression of Ig in mammalian cell lines is very laborious. Therefore more information about the antibody to be engineered is required in order to select only a few antibodies for expression in mammalian cell lines. It is also possible to express Ig's in a relatively simple E.coli expression system. Ward et al. (10) have reported the expression and secretion of VH domains (single domain antibodies, dAbs) with good antigen-binding affinity in E.coli. More promising seems the expression of Fv fragments in E.coli (11, 12). Skerra et al. (11) have described that VH and VL expressed in E.coli can assemble to a complete functional dimer (Fv) with the same affinity as the original antibody. The latter two techniques have the potential to generate a large number of 'antibodies' and hence selection may be carried out in a later stage on stable 'antibody' producing bacterial clones. Interesting antibodies may be further engineered to complete antibodies or to fusion proteins with a desired property e.g. 'antibody'-HRP conjugates.
Isolation and amplification of Ig encoding mRNA from single cells has also been reported (13, 14). However, the starting point of the PCR technique normally is the hybridoma and therefore the same problems as with the fusion techniques, namely only being able to use spleen cells, apply here.

Yet another attempt to arrive at antibody producing cells is the clonal expansion of B-cells.
The systems for cloning of B-cells have been primarily based on stimulation of B-cells with LPS (which works for murine but not for human B-cells) or with intact, cloned alloreactive T-helper cells. Recently, Zubler et al. (15, 16, 17, 18) have reported a culture system in which human and/or murine B-cells can be clonally activated in a T-cell independent manner. This system uses irradiated mutant murine EL-4 thymoma cells, EL-4/B5, in conjunction with human T-cell/macrophage supernatant as a source of proliferation and differentiation factors. The EL-4/B5 cells activate the B-cells via a MHC-nonrestricted direct cell-cell interaction. The activation signal itself is not mitogenic but sensitizes B-cells to respond to one (IL-2) or several cytokines present in human T-cell supernatant. Ninety percent of B-cells from human peripheral blood or spleen became activated and generated short term clones of a mean of 380 antibody secreting cells during a 10 days response (15). The mean amount of Ig secreted per clone was 40 ng and individual clones showed IgM, IgG, IgA, IgM + IgG or IgM + IgG + IgA production showing that Ig class switches have occurred.

Still, none of the above mentioned techniques gives the ability to arrive at stable cell lines producing any desired specific monoclonal antibody from any possible source, such as lymph node or peripheral blood of animals or from a human source.
Fusion of lymph node cells or peripheral blood cells may result in antibodies with another specificity, affinity or isotype in comparison with spleen cells and may therefore give a useful addition to the antibodies obtainable by the prior art techniques. In addition, fusion of peripheral blood cells would offer the opportunity for studying the immune repertoire of one laboratory animal during a longer period of time.

Beside monoclonal antibodies of animal origin, there is an interest for antibodies of human origin. There are several reasons for this interest:
(i) In therapeutic regimes murine monoclonal antibodies have rendered harmful to patients due to a human antimouse response (19, 20). Human monoclonals are likely to exhibit reduced or no immunogenicity in humans.
(ii) The human genome may represent another repertoire of antibodies which may consequently result in monoclonals with other specificity.
(iii) Some individuals such as immunoinfertile donors, donors with auto-immune diseases, or donors with antitumor antibodies produce antibodies of interesting specificity.

Though many investigators have reported the development of human monoclonal antibodies in recent years (21, 22), many problems in the generation of human monoclonal antibodies remain unsolved to date. A common problem in human monoclonal antibody development is that immunizations are not allowed for ethical reasons.
As a consequence almost only antibodies against naturally occurring antigens can be developed from isolated human lymphocytes. The frequency of antigen-specific B-lymphocytes will often be very low .
The results from in vitro immunization experiments with human lymphocytes have been very poor and not reproducible up till now (23, 24).
To date, the generation of human monoclonal antibodies has concentrated mainly on two methods:
(i) fusion of lymphocytes with myeloma cells of murine, human and murine x human origin and
ii) viral transformation of lymphocytes with Epstein-Barr virus (EBV).

Apart from the low fusion frequency inherent to the PEG-fusion technique (see above), fusions with human lymphocytes are hampered for the reason that the ideal fusion partner has not yet been found. Therefore the majority of human antibody producing hybridomas generated sofar has shown to be unstable with respect to growth and antibody production. In contrast to the low immortalization frequency mentioned above, human B-lymphocytes are very efficiently immortalised by EBV. In this system, however, immortalisation is restricted to a subset of B-cells resulting in monoclonal antibodies which are predominantly of the IgM class. Moreover, a part of the EBV-transformants grow poorly and attempts to fuse EBV-transformants with myeloma cells often failed due to problems with the stability of the resulting cell lines.

The invention relates to a method for producing monoclonal antibodies, comprising harvesting B-lymphocytes from a donor, subjecting said B-lymphocytes to clonal expansion by stimulation with irradiated thymoma cells, immortalizing the resulting B-lymphocytes by electrofusion with a suitable fusion partner, culturing the resulting cells and isolating the monoclonal antibodies from the culture medium.

As an intermediate for (electro)fusions or immortalizations, cell populations can be enriched for specific antibody producing cells. Thus, it may be possible to develop hybridomas from one to a few specific antibody secreting B-cells.

Preferably the procedures include a selection step for antibody producing lymphocytes (more preferably selecting for antibodies with the desired specificity) before clonal expansion and immortalization or amplification.
The selection step may also be carried out after the clonal expansion.

An important advantage of the present method lies in the combination of the clonal expansion by stimulation with irradiated thymoma cells, together with the immortalization technique by electrofusion with a suitable fusion partner.

Through the clonal expansion technique one is able to produce enough B-lymphocytes out of one to a few B-lymphocytes to allow fusion or transformation, or isolation of messenger RNA. From one lymphocyte up to five hundred lymphocytes can be grown. Thus, using the present invention antibody producing cells can be made from every possible lymphocyte source.
Another advantage of the present invention is the preselection of antibody producing lymphocytes.
Even in well-responding individuals, only a minor fraction of the lymphocytes is able to produce antibodies of desired specificity. Therefore, if immortalization techniques become more efficient, preselection of antibody producing B-cells will be a requirement in order to make optimal use of sensitized cells or to avoid laborious screening of very large numbers of supernatants resulting from electrofusion, clonal expansion of B-cells or PCR-technique on single or expanded B-cells.
Though specific selection techniques like panning, rosetting and fluorescence activated cell sorting (FACS) are well described, very little is known about whether selected cells can be used for fusion or culture. In this respect, recently developed, non-toxic, paramagnetic immunobeads seem very promising. It has been shown that after selection with paramagnetic beads, hybridoma cells could be successfully cloned and subcultivated without separation of beads and cells (25). This technique is very suitable for use as a preselection step in the present invention.
Also very useful is the preselection technique called panning.

Clonal expansion together with Electrofusion offers the possibility to fuse preselected specific B-cells. Thus the number of specific hybridomas can be significantly increased without laborious testing of negative clones. Consequently the antibody with desired properties (epitope specificity, affinity, class and subclass) can be chosen from a larger number of antibodies which increases the chance of finding very special ones.
Clonal expansion together with Electrofusion can be used to fuse small numbers of lymphocytes obtained from lymph nodes, orbita punction blood samples and in vitro immunization experiments.
Fusion of lymph node cells may result in a higher number of sensitized specific B-cells and in a greater spectrum of antibody specificities as was previously shown by Mirza et al. (26).
Fusion of peripheral blood lymphocytes has an additional advantage because the immune response can be followed during a longer period of time in the same laboratory animal.
Activated cells have an enlarged cell volume. In electrofusion larger cells are preferably fused because these cells have a smaller difference in membrane breakdown voltage in comparison with myeloma cells. Thus preferably antigen-stimulated, affinity-maturated B-cells/plasma cells are fused. This may result in more high affinity antibodies.
The combination of clonal expansion of B-cells and electrofusion offers a new method of monoclonal antibody development which has the potential to generate hybridoma cell lines from single B-cells. In this respect, the method can at least compete with the PCR technique for Ig genes. In addition it has the advantage to result in affinity matured antibodies.

As the EL-4/B-cell culture system has appeared not to be species specific (murine and human B-cells could be expanded), it is expected that also lymphocytes from other species can be cultured.
Electrofusion, PCR-technique for Ig genes and the combination of these techniques with clonal expansion of B-cells circumvents the selectivity problem (predominantly IgM antibodies) of EBV transformation in the generation of human monoclonal antibodies.
The invention will be described in more detail in the following experimental section.

### EXPERIMENTAL

### MATERIALS AND METHODS

### Reagents

- Culture medium DMEM/HAM's F12 was prepared by mixing equal amounts of Dulbecco's Modified Eagle Medium (DMEM, Gibco 074-2100) and Nutrient mixture F12 (HAM's F12, Gibco 074-1700) and supplementing this mixture with 2500 mg/l sodium bicarbonate (Baker), 55 mg/l sodium pyruvate (Fluka), 2.3 mg/l 2-mercaptoethanol (Baker), 1.22 mg/l ethanolamine (Baker), 360 mg/l L-glutamine (Merck), 4.5·10⁻⁴mg/l sodium selenite (Fluka), 62.5 mg/l sodium penicillin (Mycopharm) and 62.5 mg/l streptomycin sulphate (Serva).
   In fusion experiments, the medium was further supplemented with 13.61 mg/l hypoxanthine (Fluka) and 3.83 mg/l thymidine (Fluka). This medium is referred to as DMEM/HAM's F12/HT.
   Selection of hybridomas was performed in DMEM/HAM's F12/HT supplemented with 1% of Il-6 containing supernatant of a human bladder carcinoma cell line T24 (T24CM) and 0.4 µM aminopterin (Flow).
- Fusion medium: 280 mM inositol (ICN), 0.1 mM calcium acetate (Baker), 0.5 mM magnesium acetate (Baker) and 1 mM histidine (Fluka); specific resistance: 1.11.10⁴ Ω.cm. Ingredients were dissolved in Milli-Q water and subsequently the conductivity was adjusted to 90 µS/cm with Milli-Q water or a solution containing 1 mM calcium acetate and 5 mM magnesium acetate.
- Pronase solution was prepared by dissolving 0.5 mg/ml pronase (Calbiochem) in DMEM/HAM's F12.
- Percoll density gradient medium (Pharmacia) was made iso-osmotic by the addition of 1 part of 1.5 M NaCl to 9 parts of Percoll (100% SIP). This stock iso-osmotic Percoll solution was adjusted to lower densities by diluting with culture medium.

### Cell cultures

Mutant EL-4 thymoma cells, EL-4/B5 (a kind gift from Dr. R. Zubler, Geneva, Switzerland) were routinely cultured in DMEM/HAM's F12 supplemented with 10% FCS (Bocknek) between cell concentrations of 1.10⁴ to 1.10⁶ c/ml. If cells overgrow 1.10⁶ c/ml, they may lose their B-cell stimulating activity.
Murine myeloma cells NS-1, or xenohybrids K6H6B5 and PAI-1 were used as fusion partners for murine and human B-cells respectively. Cells were routinely cultured in DMEM/HAM's F12/HT supplemented with 10% FCS in concentrations between 5.10⁴ and 15.10⁵ cells per ml. One day before fusion, cultures were split 1:3 to create a log-phase culture at the day of fusion.

### Preparation of murine spleen cells

After final immunization, the mice were killed and their spleens removed. Cells were teased into DMEM/HAM's F12 and clumps disrupted by gentle aspiration through a 16 gauge needle. Cell suspensions were centrifuged for 10 minutes at 2000 N/kg and the pellets were resuspended in a solution containing 0.16 M NH₄Cl and 0.01 M KHCO₃ to lyse the erythrocytes. Cell suspensions were centrifuged again and finally resuspended in culture medium.

### Isolation of peripheral blood cells

Human leucocytes were isolated by bulging out the erythrocytes in an isotonic dextrane solution as described by Van Meel (1985). Subsequently the leucocytes were suspended in DMEM/HAM's F12 supplemented with 10% FCS and incubated for one hour at 37°C in polystyrene culture flasks to remove monocytes. Then, T-cells were depleted by two subsequent incubations with paramagnetic polystyrene beads coated with anti-CD2 monoclonal antibody (Dynal 111.01). For a complete depletion of CD2 positive cells, washed immunobeads and monocyte depleted PBL were mixed at a ratio of 40:1. The mixture of beads and cells was incubated for 30 min. at 2-4°C with gentle mixing every 10 min. Subsequently, rosetted cells were isolated by holding a magnetic device against the wall of the test tube and removing the supernatant.
For the isolation of murine PBL, 100 µl blood were collected by orbital punction and immediately thereafter diluted with 100 µl phosphate buffered saline (PBS) containing 30 IU/ml lithium heparin. Subsequently, the diluted blood was carefully layered on 700 µl Lymphopaque (Nyegaard & Co.) in a small capillary. Then, the capillary was centrifuged for 30 minutes at 4000 N/kg and subsequently the interphase layer was collected. Finally, platelets were removed by washing the cells twice with culture medium.

### Preparation of human T-cell/macrophage supernatant (TSN)

Freshly isolated mononuclear cells were centrifuged for 10 minutes at 2000 N/kg. Subsequently, B- and T-cells were separated according to a modification of the method described by Gutierrez et al. (1979). The pellet was resuspended in 5 ml 100% SIP. Then, a 10 ml layer of 70% SIP followed by a 25 ml layer of 50% SIP were layered onto the 100% SIP. The gradient was centrifuged for 10 min. at 25,000 N/kg. The enriched T-cell fraction remaining at the interface between 70% and 50% SIP was collected and washed twice with DMEM/HAM's F12 supplemented with 10% FCS. Washed cells were stimulated for 40-45 h in DMEM/HAM's F12 supplemented with 10% FCS, 5 µg/ml PHA (Wellcome) and 10 ng/ml PMA (Sigma).
Finally, supernatant was harvested, filtered through a 0.2 µm membrane filter and stored in aliquots at -70°C.

### EL-4/B-cell cultures

EL-4/B-cell cultures were prepared as described by Zubler et al. (12-15). Briefly, crude or purified B-cells were mixed with TSN and 50,000 irradiated (2500 RAD) EL-4/B5-cells in a final volume of 200 µl DMEM/HAM's F12 supplemented with 10% FCS in 96-well flat bottomed tissue culture plates. The optimal amount of TSN was established for each batch by titration. Usually 10% TSN was sufficient for optimal stimulation of human B-cells whereas 20% TSN was usually required for murine B-cells.
The cultures were incubated at 37°C, with 5% CO₂ and 100% humidity. Between Day 8 and Day 12, supernatants were tested for immunoglobulin production.

### Mini electrofusion

The contents of individual EL-4/B-cell cultures were mixed with 10⁶ myeloma cells in 2-ml centrifuge tubes. The cells were rendered serum-free by washing once with DMEM/HAM's F12/HT. Then, the cell suspension was centrifuged and the pellet was resuspended in 1 ml DMEM/HAM's F12/HT of 37°C and 130 µl pronase solution was added. The cell suspension was incubated for 3 minutes with pronase and then the enzyme reaction was stopped by addition of 200 µl FCS. Subsequently, the cells were washed once with fusion medium and resuspended in a final volume of 50 µl fusion medium at room temperature. The complete cell suspension was pipetted into the internal space of a fusion chamber. This chamber consists of two stainless steel, disc-shaped electrodes embedded in a perspex box. The electrodes are separated by a teflon spacer of varying diameter and 0.50 mm thickness. Alignment of cells in the fusion chamber was induced by an alternating electric field of 2 MHz and 400 V/cm for 30 s. Then, immediately a square, high field pulse of 3 kV/cm and 10 µs duration was applied causing cell membrane breakdown. The alternating field was applied again for 30 s in order to allow intermingling of cells and resealing of membranes. Finally, the contents of the fusion chamber were transferred to 20 ml selection medium and plated into a 96-well microculture plate.
At Day 14, the cultures were examined for hybridoma growth and the supernatants were tested for immunoglobulin production.

### Enzyme immunoassays

Measurement of human Ig was performed by a standard sandwich ELISA using goat anti-human Ig coated plates and HRP-labeled goat anti-human Ig second antibody. Murine Ig was measured with a similar ELISA employing sheep anti-mouse antibodies. For the detection of murine anti-HIV antibodies, plates coated with HIV viral lysate were used in combination with HRP-labeled sheep anti-mouse Ig. Human anti-rubella antibodies were detected in a double sandwich ELISA with goat anti-human Ig as the capture antibody and rubella antigen bound to murine monoclonal anti-rubella-HRP.

### Panning procedure.

Six-well culture plates were incubated overnight with 4 ml per well of a solution containing 1 to 10 ug antigen in 0.05 M sodiumcarbonate buffer pH=9.6. Subsequently, the wells were washed with PBS and directly used for panning experiments or stored at -20 °C.
Panning was performed by incubating monocyte depleted murine spleen cells or human mononuclear cells on antigen coated wells for 1 to 2 hour at 37 ^{o}C, 5% CO₂ and 100% humidity. After this incubation, the unattached cells were removed gently by three subsequent washes with PBS. Then, the antigen-bound, specific B-cells were recovered by incubating each well with 250 ul PBS containing 1.1 mM Na₂EDTA and 0.05 % trypsin (Flow, cat no. 16-893-49) pH=7.5 for 2 minutes. Trypsin treatment was stopped by addition of 5 ml DMEM/HAM's F12 supplemented with 10% FCS. Finally, the entire surface of the wells was flushed with the medium using a pasteur pipette in order to remove residual attached B-cells mechanically.

### RESULTS

### - Immortalization of murine B-cells

In two independent experiments, EL-4/B-cell cultures were inoculated with murine spleen cells or mononuclear peripheral blood cells at seeding densities of 50, 10 or 2 cells per well. Supernatants of the cultures were tested (day 8-11) for the presence of murine Ig, indicating outgrowth of murine B-cells. Table I shows that an outgrowth of B-cells had been achieved in approximately 50 percent of cultures inoculated with 2 spleen cells and in approximately 20 percent of cultures inoculated with 2 mononuclear peripheral blood cells.

Twelve individual cultures were submitted to mini-electrofusions with NS-1 myeloma cells in order to investigate whether it would be possible to generate antibody-producing hybridomas from just a small number of B-cells present in B-cell cultures. Table II shows, that a large number of antibody producing hybridomas were generated in 11 out of 12 fusion experiments.

We further investigated whether it would be possible to develop antigen specific hybridomas. Therefore, spleen cells and peripheral blood mononuclear cells were isolated from mice immunized with HIV viral lysate. At Day 9, the cultures were examined for cell growth and the supernatants were assayed for murine immunoglobulin and murine anti-HIV by ELISA. Table III shows that outgrowth of Ig-producing B-cells occurred in 52 percent of cultures inoculated with 2 spleen cells and in 35 percent of cultures inoculated with 4 peripheral blood cells. No anti-HIV producing wells were found in these cultures, however in cultures inoculated at higher seeding densities 3 and 4 anti-HIV producing B-cell clones were found in spleen and PBL cultures, respectively. All anti-HIV producing cultures were individually submitted to mini-electrofusions with NS-1 myeloma cells in order to convert the anti-HIV B-cell clones into continuous growing hybridoma cell lines. In five out of seven mini-electrofusions at least one antigen-specific hybridoma was generated (Table IV).

B-cell clones in cultures inoculated with 2 spleen cells or 4 PBL were expected to originate from single B-cells. Therefore two additional mini-electrofusions were performed with these cultures to investigate whether it would be possible to develop antibody-producing hybridomas from B-cell cultures inoculated with single B-cells. These fusions resulted in 45 and 75 Ig-producing hybridomas, respectively (results not shown).

**Table I**

| Outgrowth of murine B-cells | | | |
|---|---|---|---|
| Murine spleen cells or peripheral blood lymphocytes were inoculated in 96-well culture plates in the presence of 50,000 irradiated EL-4/B5 helper cells and 20% human T-cell supernatant. Culture supernatants were tested for murine Ig from Days 8 to 11. | | | |
| cell type isolated | number of wells seeded | number of cells per well | number of Ig positive wells |
| spleen | 48 | 50 | 48 |
| PBL | 48 | 50 | 45 |
| spleen | 232 | 10 | 217 |
| PBL | 136 | 10 | 78 |
| spleen | 144 | 2 | 74 |
| PBL | 48 | 2 | 8 |

**Table II**

| Mini-electrofusions of B-cell cultures | | | | |
|---|---|---|---|---|
| Murine B-cell cultures from the experiment described in Table I were mixed with 10⁶ NS-1 myeloma cells. Cells were pronase-treated and finally suspended in fusion medium. After fusion, cells were seeded in 96-well culture plates in selection medium. Nine days after fusion, cultures were examined for hybridoma growth and supernatants were tested for murine Ig. | | | | |
| Fusion no. | origin of B-cell culture | number of wells plated in fusion | number of wells with hybridoma growth | number of Ig-positive wells |
| 1 | 50 spleen cells | 48 | 48 | 48 |
| 2 | 50 spleen cells | 48 | 48 | 48 |
| 3 | 50 PBL | 48 | 47 | 46 |
| 4 | 50 PBL | 48 | 43 | 42 |
| 5 | 10 spleen cells | 48 | 40 | 37 |
| 6 | 10 spleen cells | 48 | 47 | 47 |
| 7 | 10 spleen cells | 96 | 96 | 96 |
| 8 | 10 PBL | 48 | 48 | 48 |
| 9 | 10 PBL | 48 | 3 | 2 |
| 10 | 2 spleen cells | 96 | 94 | 92 |
| 11 | 2 spleen cells | 96 | 78 | 78 |
| 12 | 2 spleen cells | 96 | 96 | 96 |

**Table III**

| Outgrowth of anti-HIV specific murine B-cells in EL-4/B-cell cultures | | | | |
|---|---|---|---|---|
| Murine spleen cells or peripheral blood lymphocytes were inoculated in 96-well culture plates in the presence of 50,000 irradiated (2500 RAD) EL-4/B5 helper cells and 20% human T-cell supernatant. At Day 9, culture supernatants were tested for murine Ig or murine anti-HIV antibodies. | | | | |
| Cell type isolated | number of wells seeded | number of cells per well | number of Ig-positive wells | number of anti-HIV positive wells |
| spleen | 190 | 10 | n.t.*) | 3 |
| spleen | 190 | 2 | 99 | 0 |
| PBL | 190 | 20 | n.t. | 4 |
| PBL | 190 | 4 | 66 | 0 |

| | | | | |
|---|---|---|---|---|
| *) n.t. = not tested | | | | |

**Table IV**

| Mini-electrofusion of individual anti-HIV positive B-cell cultures | | | | |
|---|---|---|---|---|
| Murine B-cell cultures from the experiment described in Table III were mixed with 10⁶ NS-1 myeloma cells. Cells were pronase-treated and finally resuspended in fusion medium. After fusion, cells were seeded in 96-well culture plates in selection medium. Thirteen days after fusion, cultures were examined for hybridoma growth and supernatants were tested for murine Ig and murine anti-HIV. | | | | |
| Fusion no. | number of wells seeded | number of wells with hybridoma growth | number of Ig-positive wells | number of anti-HIV positive wells |
| 1 | 96 | 96 | 96 | 1 |
| 2 | 96 | 72 | 65 | 7 |
| 3 | 96 | 96 | 96 | 3 |
| 4 | 96 | 42 | 40 | 7 |
| 5 | 96 | 30 | n.t. *) | 0 |
| 6 | 96 | 11 | 11 | 0 |
| 7 | 96 | 6 | 2 | 2 |

| | | | | |
|---|---|---|---|---|
| *) n.t. = not tested | | | | |

### - Immortalization of human B-cells

From a donor who had previously suffered a rubella infection, human leucocytes were isolated as described in the 'Materials and methods' section. FACS-analysis showed that the final preparation contained approximately 11 percent B-cells, 4 percent monocytes and 1 percent T cells. This cell population was used to inoculate EL-4/B-cell cultures. Cultures were seeded with 850, 250, 25, 50 and 10 cells per well representing a mean of 90, 25, 5, 2.5, and 1 B-cell per well, respectively. Eight days after inoculation, human Ig was measured in 43 percent of cultures, inoculated with one B-cell. No anti-rubella positive clones were detected in these cultures, but eighteen anti-rubella positive clones were found in cultures inoculated with more B-cells. (Table V). These cultures were submitted separately to mini-electrofusions with K6H6B5 or PAI-1 myeloma cells (Table VI). In all fusion experiments a large number of hybridomas was generated. However, only a small number of these hybridomas produced immunoglobulin. Initially, in three fusions, anti-rubella positive hybridomas could be detected. From one fusion, a hybridoma was scaled up and cloned to a stable anti-rubella IgG producing cell line. From another fusion, two anti-rubella IgM hybridomas were generated which were also successfully scaled up and cloned. The anti-rubella positive clones from the third fusion were lost. They were probably overgrown by the relatively large number of non-producing hybridomas.

**Table V**

| Outgrowth of human anti-rubella B-cells in EL-4/B-cell cultures | | | |
|---|---|---|---|
| Monocyte and T-cell depleted human leucocytes were inoculated in 96-well culture plates in the presence of 50,000 irradiated (2500 RAD) EL-4/B5 helper cells and 20% human T-cell supernatant. At Day 8, culture supernatants were tested for human Ig and human anti-rubella antibodies. | | | |
| mean number of B-cells per well | number of wells seeded | number of Ig positive wells | number of anti-rubella positive wells |
| 90 | 87 | 87 | 3 |
| 25 | 698 | 698 | 12 |
| 5 | 190 | 146 | 1 |
| 2.5 | 410 | 361 | 2 |
| 1 | 190 | 82 | 0 |

**Table VI**

| Mini-electrofusion of individual human B-cell cultures | | | | | |
|---|---|---|---|---|---|
| Human B-cell cultures from the experiment described in Table V were mixed with 10⁶ K6H6B5 or 10⁶ PAI-1 myeloma cells. Cells were pronase-treated and finally resuspended in fusion medium. After fusion, cells were seeded in 96-well culture plates in selection medium. Fifteen days after fusion, cultures were examined for hybridoma growth and supernatants were tested for human Ig and anti-rubella. | | | | | |
| Fusion no | fusion partner | number of wells seeded | number of wells with hybridoma growth | number of Ig-positive wells | number of anti-rubella positive wells |
| 1 | K6H6B5 | 96 | 95 | 2 | 0 |
| 2 | K6H6B5 | 96 | 80 | 1 | 0 |
| 3 | K6H6B5 | 96 | 75 | 7 | 0 |
| 4 | K6H6B5 | 96 | 24 | 0 | 0 |
| 5 | K6H6B5 | 96 | n.d. | 1 | 0 |
| 6 | K6H6B5 | 96 | n.d. | n.d. | 2 |
| 7 | K6H6B5 | 96 | n.d. | n.d. | 0 |
| 8 | K6H6B5 | 96 | n.d. | n.d. | 0 |
| 9 | K6H6B5 | 96 | n.d. | n.d. | 0 |
| 10 | K6H6B5 | 96 | n.d. | n.d. | 0 |
| 11 | PAI-1 | 96 | 87 | 3 | 0 |
| 12 | PAI-1 | 96 | 33 | 0 | 0 |
| 13 | PAI-1 | 96 | n.d. | 6 | 1 |
| 14 | PAI-1 | 96 | n.d. | n.d. | 0 |
| 15 | PAI-1 | 96 | n.d. | n.d. | 0 |
| 16 | PAI-1 | 96 | n.d. | n.d. | 0 |
| 17 | PAI-1 | 96 | n.d. | n.d. | 0 |
| 18 | PAI-1 | 96 | n.d. | n.d. | 0 |
| n.d. = not determined | | | | | |

### Selection example 1

In two independent experiments, leucocytes from a human donor that previously had undergone a rubella infection were isolated by bulging out the erythrocytes in an isotonic dextrane solution. Subsequently, the leucocytes were incubated for two hour in polystyrene culture flasks in order to remove monocytes.
Then, 3 ml of the unattached cell population was submitted to a panning on a rubella coated well. In this way selected cells (cell population B) were used to inoculate EL-4/B-cell cultures at different cell concentrations. As a control, 0.7 ml of the unattached cell population was depleted from T- an NK-cells by treatment with anti-CD2 and anti-CD16 paramagnetic immunobeads respectively. These cells (cell population A) were also used to inoculate EL-4/B-cell cultures.
At day 11, the B-cell cultures were tested for the presence of human anti-rubella antibodies and total human Ig. Table VII shows that panning resulted in a substantial enrichment of antigen-specific B-cells in both experiments. In experiment I, up to 1 out of 5 antibody producing B-cell clones were anti-rubella specific and 1 out of 3 in experiment II. Further, it was established that the major part of the antigen specific B-cells were IgG producers.

**Table VII**

| **Human anti-rubella** | | | | | |
|---|---|---|---|---|---|
| Exp. no. | cell type | number of B-cell cultures | estimated number of B-cells per well | Ig positive cultures | rubella positive cultures |
| I | A | 95 | 66 | n.d. | 3 |
| | A | 95 | 22 | n.d. | 3 |
| | A | 95 | 5 | n.d. | 0 |
| | A | 95 | 5 | 89 | 2 |
| | B | 95 | (5) | 88 | 30 |
| | B | 95 | (1) | 35 | 6 |
| | B | 95 | (0,3) | 11 | 2 |
| II | A | 95 | 72 | n.d. | 2 |
| | A | 95 | 24 | n.d. | 2 |
| | A | 95 | 5 | n.d. | 0 |
| | A | 95 | 5 | 95 | 0 |
| | B | 95 | (4) | 62 | 26 |
| | B | 95 | (1) | 26 | 8 |
| | B | 95 | (0,3) | 9 | 0 |
| A : T-cell and monocyt depleted leucocytes (approx. 10 % B-cells) B : Cells selected on rubella coated dishes n.d. = not determined | | | | | |

### Selection example 2

Peripheral blood lymphocytes from a chimpanzee that was immunized with cytomegalovirus (CMV) were isolated on Ficoll-paque. Subsequently, these lymphocytes were incubated for two hour in polystyrene culture flasks in order to remove monocytes.
Then, 12.10⁵ cells of the unattached cell population were submitted to a panning on a CMV coated well. In this way selected cells (cell population B) were used to inoculate EL-4/B-cell cultures at different cell concentrations. As a control, 2.4.10⁵ cells of the unattached cell population were depleted from T- an NK-cells by treatment with anti-CD2 and anti-CD16 paramagnetic immunobeads respectively. These cells (cell population A) were also used to inoculate EL-4/B-cell cultures.
At day nine, the B-cell cultures were tested for the presence of anti-CMV antibodies and total chimpanzee Ig. Table VIII shows that, though a large number of B-cells were inoculated, no anti-CMV positive wells were found in the B-cell cultures inoculated with unselected cells (cell population A). On the contrary, two anti-CMV positive wells were found in B-cell cultures inoculated with a relatively low amount of selected cells. This example stresses that panning may be a very usefull tool to select antigen specific B-cells from large cell populations with a low amount of antigen specific B-cells because otherwise too many samples have to be screened.

**Table VIII**

| **Chimpanzee anti-CMV** | | | | | |
|---|---|---|---|---|---|
| exp. no. | cell type | number of B-cell cultures | estimated number of B-cell per well | Ig positive cultures | anti-CMV positive cultures |
| I | A | 95 | 330 | n.d. | 0 |
| | A | 95 | 110 | n.d. | 0 |
| | A | 95 | 20 | 51 | 0 |
| | A | 95 | 10 | 26 | 0 |
| | B | 95 | (4) | 80 | 2 |
| | B | 95 | (1) | 43 | 0 |
| | B | 95 | (0,5) | 22 | 0 |
| A : T-cell, NK-cell and monocyt depleted mononuclear cells (approx. 80 % B-cells) B : Cells selected on CMV-coated dishes ( ):Cloning-efficiencies are usually between 25 and 75 percent. These figures are based on a cloning-efficiency of 30 percent. n.d. = not determined | | | | | |

### Selection example 3

Peripheral blood lymphocytes from a human donor, that had undergone a primary CMV infection 11 weeks before, were isolated on Ficoll-paque. Subsequently, the lymphocytes were incubated overnight in a polystyrene culture flask in order to remove monocytes.
Then, 18.10⁵ cells of the unattached cell population were submitted to a panning on a CMV coated well. In this way selected cells (cell population B) were used to inoculate EL-4/B-cell cultures at different cell concentrations. As a control, 4.10⁵ monocyte depleted cells were depleted from T- an NK-cells by treatment with anti-CD2 and anti-CD16 paramagnetic immunobeads respectively. These cells (cell population A) were also used to inoculate EL-4/B-cell cultures.
At day nine, the B-cell cultures were tested for the presence of anti-CMV antibodies and total human Ig. Table IX shows that panning resulted in a substantial enrichment of antigen-specific B-cells. After panning, up to 1 out of 2 antibody producing B-cell clones were anti-CMV specific. Table IX also shows that the major part of the antigen specific B-cells were IgG producers.

**Table IX**

| **Human anti-CMV** | | | | | | |
|---|---|---|---|---|---|---|
| exp. no. | cell type | number of B-cell cultures | estimated number of B-cell per well | Ig positive cultures | anti-CMV positive cultures | Ig-class IgG:IgM: IgM+IgG |
| I | A | 95 | 350 | n.d. | 19 | |
| | A | 95 | 60 | n.d. | 4 | |
| | A | 95 | 12 | 76 | 1 | |
| | B | 95 | (0,4) | 10 | 4 | 34:1:1 |
| | B | 95 | (0,2) | 6 | 2 | |
| A : T-cell, NK-cell and monocyt depleted mononuclear cells (approx. 80 % B-cells) B : Cells selected on CMV-coated dishes ( ) : Cloning-efficiencies are usually between 25 and 75 percent. These figures are based on a cloning-efficiency of 30 percent. n.d. = not determined | | | | | | |

### Selection example 4

Spleen cells from a mouse that was immunized with HIV viral lysate were isolated as described above. Subsequently, these spleen cells were incubated in a polystyrene culture flask for 1.5 hour in order to remove monocytes.
Then, 175.10⁵ unattached cells were submitted to a panning on five HIV viral lysate coated wells. In this way selected cells (cell population B) were used to inoculate EL-4/B-cell cultures. Control EL-4/B-cell cultures were inoculated with unselected, monocyte depleted spleen cells (cell population A).
At day nine, the B-cell cultures were tested for the presence of anti-HIV antibodies and total murine Ig. Table X shows that no antigen specific B-cell clones were found in B-cell cultures inoculated with unselected cells whereas 6 antigen specific B-cell clones were found in cultures inoculated with selected cells.

**Table X**

| Cell population | number of B-cell inoculated | number of Ig producing B-cell cultures | number of anti-HIV producing B-cell cultures |
|---|---|---|---|
| A | 285 | 78 | 0 |
| B | 285 | 75 | 6 |

### Selection example 5

In order to generate monoclonal antibodies to human ZP3, mice were immunized with recombinant fusion-protein βGAL-ZP3. However, on account of the strong immunogenicity of βGAL, a large response against βGAL was obtained and the response against ZP3 was very weak. For this reason, only one anti-ZP3 monoclonal antibody has been developed from a great number of electrofusions with unselected spleen cells.
In this example, the majority of anti-βGAL producing B-cells were first removed by absoption on βGAL coated wells. Subsequently, a panning on βGAL-ZP3 coated wells was performed in order to select anti-ZP3 producing B-cells. Finallly, selected cells were expanded in EL-4/B-cell cultures and immortalized by mini-electrofusions with NS-1 myeloma cells.

Spleen cells from a mouse that was immunized with recombinant βGAL-ZP3 were isolated as described above. Subsequently, these spleen cells were incubated in a polystyrene culture flask for 1.5 hour in order to remove moncytes.
Then, 17.10⁶ unattached cells were incubated for 1.5 hour at 37 °C, 5% CO₂ and 100% humidity on four βGAL coated wells. After this incubation, the nonadherent cells were submitted to a panning on four βGAL-ZP3 coated wells. In this way selected cells (cell population B) were used to inoculate EL-4/B-cell cultures. Control EL-4/B-cell cultures were inoculated with unselected, monocyte-depleted spleen cells (cell population A).
At day eight, the B-cell cultures were tested for the presence of anti-βGAL antibodies, anti-βGAL-ZP3 antibodies and total murine Ig. Table XI shows that despite the absorption of anti-βGAL producing B-cells, still a large number of anti-βGAL positive B-cell clones were found among the selected cells. However, there were also found two B-cell cultures that produce antibodies which discriminate between βGAL and βGAL-ZP3.

**Table XI**

| exp. no. | cell population | number of wells | number of cells per well | number of wells Ig-pos. | number of wells anti-βGAL positive | number of wells anti-βGAL ZP3 pos. |
|---|---|---|---|---|---|---|
| I | A | 95 | 50 | n.t. | 19 | 19 |
| II | A | 95 | 50 | n.t. | 19 | 19 |
| III | A | 95 | 10 | 95 | 13 | 13 |
| IV | B | 95 | 80 | 95 | 86 | 87 |
| V | B | 95 | 20 | 95 | 32 | 33 |
| VI | B | 95 | 10 | 95 | 34 | 34 |
| n.t. = not tested A: monocyte depleted mouse mile cells Muly 59 B: On βGAL-ZP3 selected cells which were first depleted on βGAL coated wells | | | | | | |

Six anti-βGAL positive B-cell clones and the two clones that discriminate between βGAL-ZP3 and βGAL were submitted to mini-electrofusions with 10⁶ NS-1 cells. Eight days after this fusion, the hybridoma cultures were tested for the production of anti-βGAL and anti-βGAL-ZP3 antibodies. Table XII shows that all B-cell clones were succesfully converted into specific antibody producing hybridomas. Moreover, in one fusion also two hybridomas were generated that recognize βGAL-ZP3 and did not recognize βGAL.

**Table XII**

| exp. no. | number of wells | number of wells with hybridomas | number of anti-βGAL positive | number of anti-βGAL ZP3 pos. |
|---|---|---|---|---|
| I | 95 | 95 | 45 | n.t. |
| II | 95 | 95 | 4 | 6 |
| III | 95 | 95 | 8 | 8 |
| IV | 95 | 95 | 6 | 6 |
| V | 95 | 95 | 66 | n.t. |
| VI | 95 | 95 | 6 | n.t. |
| VII | 95 | 95 | 12 | 12 |
| VIII | 95 | 95 | 9 | n.t. |
| n.t. = not tested | | | | |

### DISCUSSION

Limiting dilution experiments have shown a very efficient outgrowth of murine B-cells originating from spleen and peripheral blood in cultures with EL-4/B5 helper cells and human T-cell supernatant. Murine Ig production was found in 40 to 60 percent of cultures inoculated with as little as 2 cells from a crude spleen cell population. About a twofold lower amount of Ig producing cultures was found among cultures inoculated with murine PBL which is in agreement with the lower percentage of B-cells in peripheral blood. Mini-electrofusions of such B-cell cultures, which were expected to originate from one B-cell, always yielded a large number of antibody producing hybridomas.
The method has been applied successfully to the generation of murine anti-HIV producing hybridomas. Murine anti-HIV producing B-cells were expanded in cultures inoculated with 10 spleen cells or 20 PBL. Five out of seven anti-HIV producing cultures could be converted into anti-HIV producing hybridomas. The two failures may be due the presence of a majority of non-specific B-cells in the oligoclonally expanded B-cell cultures. Unfortunately, no anti-HIV producing clones were obtained from clonally expanded B-cell cultures inoculated with 2 spleen cells or 4 PBL. The latter might be due to the low number of antigen-specific B-cells in the crude cell population used in relation to the number of wells seeded. Not only its high efficiency but in particular the possibility to use small numbers of lymphocytes from murine PBL or lymph nodes gives the method a new dimension to the development of murine MAb. Fusion of lymph node cells may result in a higher number of sensitized specific B-cells and a greater spectrum of antibody specificities as was previously shown by Mirza et al. (1987). Fusion of PBL obtained from orbital punctions allows monitoring of the immune response for a longer period of time in the same animal at the monoclonal level.
As the B-cell culture system can also be used for the expansion of human B-cells (Wen et al., 1987; Zubler et al., 1987; Straub and Zubler, 1989; Zhang et al., 1990) we investigated, whether this novel method could also be applied to the development of human MAb. It appeared, that the outgrowth of B-cells was again very efficient. Mini-electrofusion of individual B-cell cultures resulted in a large number of hybridomas, though only a small proportion were Ig-producers.
One might argue, that this is due to the instability of hybridomas formed from human B-cells and the xenohybrid fusion partners (K6H6B5 and PAI-1). However, there are several indications, that this is not the case. Other experiments in our laboratory have shown that the majority of hybridomas from EBV-transformed B-cells and the xenohybrids were stable antibody producers for more than three months. Furthermore, microscopic examination of B-cell cultures inoculated with 10 cells per well of a cell population containing approximately 11 percent B-cells revealed that cell growth in these wells was not very well correlated with Ig production. In some cultures, many cells had grown out and no Ig production was detected whereas in other cultures hardly any cells were visible between the EL-4/B5 helper cells and a significant amount of Ig was measured. Apparently the size of B-cell clones was small and other cells grew in the B-cell culture system e.g. NK-cells and residual T-cells. This may be an explanation for large number of non-producing hybridomas (hybridomas from cells other than B-cells).
Notwithstanding the lower immortalization efficiency of the new method for human B-cells as compared to murine B-cells, two different human anti-rubella producing hybridomas from as little as eighteen antigen specific B-cell clones were generated. We are now optimizing the B-cell culture system in order to enlarge the clone size to a level that would guarantee at least a small number of antigen specific hybridomas from mini-electrofusions. The method described here may represent an attractive alternative for the generation of human MAb. Its high efficiency makes the method superior with respect to classical hybridoma technology and enables the use of the small numbers of lymphocytes available from human donors. It may also be possible to handle small numbers of specific lymphocytes left over from positive selection procedures, thereby avoiding laborious screening of negative hybridomas. Furthermore, immortalization of B-cells in this method may not be restricted to a subset of B-cells and therefore can result in more IgG producing cell lines than the EBV-transformation technique.

### REFERENCES

1. Kohler, G., Milstein, C. (1975). Continuous cultures of fused cells secreting antibody of predefined specificity. Nature 256, 495-497.
(2). Zimmermann, U. (1982). Electric field-mediated fusion and related electrical phenomena. Biochemical and Biophysical Acta 694, 227.
3. Van Duijn, G., Langedijk, J.P.M., De Boer, M., Tager, J.M. (1989). High yield of specific hybridomas obtained by electrofusion of murine lymphocytes immunized in vivo or in vitro. Experimental Cell Research 183, 463-472.
4. Saiki, R.K., Scharf, S., Faloona, F., Mullis, K.B., Horn, G.T., Ehrlich, H.A., Arnheim, N. (1985). Enzymatic amplification of β-globin genomic sequences and restriction site analysis for diagnosis of sickle cell anemia. Science 230, 1350-1354.
5. Orlandin, R., Güssow, D.H., Jones, P.T., Winter, G. (1989). Cloning immunoglobulin variable domains for expression by the polymerase chain reaction. Proc. Natl. Acad. Sci. USA 86, 3833-3837.
6. Larrick, J.W., Danielsson, L., Brenner, C.A., Abrahamson, M., Fry, K.E., Borrebaeck, C.A.K. (1989). Rapid cloning of rearranged immunoglobulin genes from human hybridoma cells using mixed primers and the polymerase chain reaction. Biochemical and Biophysical Research Communications 160, 1250-1256.
7. Larrick, J.W., Danielsson, L., Brenner, C.A., Wallace, E.F., Abrahamson, M., Fry, K.E., Borrebaeck, C.A.K. (1989). Polymerase chain reaction using mixed primers: cloning of human monoclonal antibody variable region genes from single hybridoma cells. Bio/technology 7, 934-938.
8. Chiang, Y.L., Sheng-Dong, R., Brow, M.A., Larrick, J.W. (1989). Direct cDNA cloning of the rearranged immunoglobulin variable region. BioTechniques 7, 360-366.
9. LeBoeuf, R.D., Galin, F.S., Hollinger, S.K., Peiper, S.C., Blalock, J.E. (1989). Cloning and sequencing of immunoglobulin variable region genes using degenerate oligodeoxyribonucleotides and polymerase chain reaction. Gene 82, 371-377.
10. Ward, E.S., Güssow, D., Griffiths, A.S., Jones, P.T., Winter, G. (1989). Binding activities of a repertoire of single immunoglobulin variable domains secreted from E.coli. Nature 341, 544-546.
11. Skerra, A., Plückthun, A. (1988). Assembly of a functional Fv fragment in Escherichia coli. Science 240, 1038-1040.
12. Better, M., Chang, C.P., Robinson, R.R., Horwitz, A.H. (1988). Escherichia coli secretion of an active chimeric antibody fragment. Science 240, 1041-1043.
13. Larrick, J.W., Danielsson, L., Brenner, C.A., Wallace, E.F., Abrahamson, M., Fry, K.E., Borrebaeck, C.A.K. (1989). Polymerase chain reaction using mixed primers: cloning of human monoclonal antibody variable region genes from single hybridoma cells. Bio/technology 7, 934-938.
14. Li, H., Gyllensten, U.B., Cui, X., Saiki, R.K., Erlich, H.A., Arnheim, N.. Amplification and analysis of DNA sequences in single human sperm and diploid cells. Nature 335, 414-417.
15. Zubler, R.H., Werner-Favre, C., Wen, L., Sekita, K., Straub, C. (1987). Theoretical and practical aspects of B-cell activation: Murine and human systems. Immunological Reviews 99, 281-197.
16. Wen, L., Hanvanich, M., Werner-Favre, C., Brouwers, N., Perrin, L.H., Zubler, R.H. (1987). Limiting dilution assay for human B-cells based on their activation by mutant EL-4 thymoma cells: total and anti-malaria responder B-cell frequencies. European Journal of Immunology 17, 887-897.
17. Werner-Favre, C., Vischer, T.L., Wohlwend, D., Zubler, R.H. (1989). Cell surface antigen CD5 is a marker for activated human B-cells. European Journal of Immunology 19, 1209-1213.
18. Straub, C., Zubler, R.H. (1989). Immortalisation of EBV-infected B-cells is not influenced by exogenous signals acting on B-cell proliferation. The journal of Immunology 142, 87-93.
19. Shawler, D.L., Bartholomew, R.M., Smith, L.M., Dillman, R.D. (1985). Human immune response to multiple injections of murine IgG. Journal of Immunology 135, 1530-1535.
20. Miller, R.A., Oseroff, A.R., Stratte, P.T., Levy, R. (1983). Blood 62, 988-995.
21. Masuho, Y. (1988). Human monoclonal antibodies: prospects for use as passive immunotherapy. Serodiagnosis and immunotherapy in Infectious disease 2, 319-340.
22. James, K., Bell, G.T. (1987). Human monoclonal antibodies; current status and future prospects. Journal of Immunological Methods 100, 5-40.
23. Borrebaeck, C.A.K. and Möller, S.A. (1986). Journal of Immunology 136, 3710-3715.
24. Borrebaeck, C.A.K. (1989). Strategy for the production of human monoclonal antibodies using in vitro activated B-cells. Journal of Immunological Methods, 123, 157-165.
25. Ossendorp, F., Bruning, P., Van den Brink, J., De Boer, M. (1989). Efficient selection of high affinity B-cell hybridomas using antigen coated magnetic beads. Journal of Immunological Methods 120, 191-200.
26. Mirza, I.H., Wilkin, T.J., Cantarini, M., Moore, K. (1987). A comparison of spleen and lymph node cells as fusion partners for the raising of monoclonal antibodies after different routes of immunization. Journal of Immunological Methods 105, 235-243.

## Claims

1. A method for producing monoclonal antibodies, comprising the steps of:
1. harvesting B-lymphocytes from a donor;
2. subjecting said B-lymphocytes to clonal expansion by stimulation of said lymphocytes with irradiated thymoma cells;
3. immortalizing said cloned B-lymphocytes by electrofusion with a suitable fusion partner;
4. culturing the resulting hybridoma cells in a culture medium; and
5. isolating the monoclonal antibodies from the culture medium.

2. A method according to claim 1 characterized in that the irradiated thymoma cells are irradiated murine EL-4/B5 thymoma cells.

3. A method according to claim 1 or 2 characterized in that the B-lymphocytes are stimulated with the irradiated murine thymoma cells in combination with human T-cell/macrophage supernatant.

4. A method according to any of the claims 1-3 characterized in that the clonal expansion of the B-lymphocytes is either preceded or followed by selection of the B-lymphocytes which produce antibodies with the desired specificity.

5. A method according to any of the claims 1-4 characterized in that one or more interleukins are used in combination with the irradiated thymoma cells.

## Patentansprüche

1. Verfahren zur Herstellung monoklonaler Antikörper, umfassend die folgenden Schritte:
1. Abtrennen der B-Lymphozyten eines Spenders;
2. Durchführen einer klonalen Expansion mit den genannten B-Lymphozyten mittels Stimulation dieser Lymphozyten mit bestrahlten Thymustumorzellen;
3. Immortalisieren der genannten geklonten B-Lymphozyten durch Elektrofusion mit einem geeigneten Fusionspartner;
4. Züchten der erhaltenen Hybridomazellen in einem Kulturmedium; und
5. Isolieren der monoklonalen Antikörper aus dem Kulturmedium.

2. Verfahren nach Anspruch 1, dadurch gekennzeichnet, dass die bestrahlten Thymustumorzellen bestrahlte EL-4/B5 Maus-Thymustumorzellen sind.

3. Verfahren nach Anspruch 1 oder 2, dadurch gekennzeichnet, dass die B-Lymphozyten mit den bestrahlten Maus-Thymustumorzellen in Kombination mit einem menschlichen T-Zellen/Makrophagen-Überstand stimuliert werden.

4. Verfahren nach einem der Ansprüche 1 bis 3, dadurch gekennzeichnet, dass der klonalen Expansion der B-Lymphozyten die Wahl der B-Lymphozyten, die Antikörper mit der gewünschten Spezifität produzieren, entweder vorangeht oder nach-folgt.

5. Verfahren gemäss einem der Ansprüche 1 bis 4, dadurch gekennzeichnet, dass ein oder mehrere Interleukine in Kombination mit den bestrahlten Thymustumorzellen verwendet werden.

## Revendications

1. Procédé pour la production d'anticorps monoclonaux, comprenant les étapes de :
1. récolte de lymphocytes B à partir d'un donneur;
2. soumission de ces lymphocytes B à une expansion clonale par stimulation de ces lymphocytes avec des cellules de thymome irradiées;
3. immortalisation de ces lymphocytes B clonés par électrofusion avec un partenaire de fusion convenable;
4. culture des cellules d'hybridome résultantes dans un milieu de culture; et
5. isolement des anticorps monoclonaux à partir du milieu de culture.

2. Procédé suivant la revendication 1, caractérisé en ce que les cellules de thymome irradiées sont des cellules murines irradiées de thymome EL-4/B5.

3. Procédé suivant les revendications 1 ou 2, caractérisé en ce que les lymphocytes B sont stimulés avec les cellules de thymome irradiées en combinaison avec un surnageant de cellules T humaines/macrophages humains.

4. Procédé suivant l'une quelconque des revendications 1 à 3, caractérisé en ce que l'expansion clonale des lymphocytes B est soit précédée, soit suivie d'une sélection des lymphocytes B qui produisent des anticorps dotés de la spécificité désirée.

5. Procédé suivant l'une quelconque des revendications 1 à 4, caractérisé en ce qu'une ou plusieurs interleukines sont utilisées en combinaison avec les cellules de thymome irradiées.
